# EUROPEAN PATENT SPECIFICATION

(11) **EP 3 986 295 B1**
(45) Date of publication and mention of the grant of the patent: **04.12.2024**
(21) Application number: 20761072.6
(22) Date of filing: 11.08.2020
(51) Int. Cl.: A61B 17/3203, A61B 18/14

(54) **SYSTEMS FOR A DISSECTION TOOL**
SYSTEME FÜR EIN SEZIERWERKZEUG
SYSTÈMES POUR UN OUTIL DE DISSECTION

(30) Priority: 15.08.2019 US 201962887230 P
(43) Date of publication of application: 27.04.2022
(60) Divisional application: 24205364.3
(73) Proprietor: Boston Scientific Scimed, Inc., Maple Grove, MN 55311-1566 (US)
(72) Inventor: RICHARDS, Laura Emily, Worcester, MA 01609 (US); KILLINGER, Kyla, Denver, CO 80238 (US); LINNABARY, Kirsten, Gahanna, OH 43230 (US)
(74) Representative: Vossius & Partner Patentanwälte Rechtsanwälte mbB
(86) International application number: PCT/US2020/045688
(87) International publication number: WO 2021/030304

(56) References cited:
- US-A- 4 898 574
- US-A- 5 944 686
- US-A1- 2006 206 028
- US-A1- 2012 221 027
- US-A1- 2013 158 544
- US-A1- 2015 112 188
- US-A1- 2018 206 864

## Description

### TECHNICAL FIELD

The present disclosure relates generally to medical devices, including endoscopic devices for tissue resection. In particular, embodiments of the claimed invention relate to medical devices for fluid powered endoscopic dissection.

### BACKGROUND

Tumor resection and other tissue treatment is often performed by medical devices (e.g., endoscopic devices) by delivering radio frequency (RF) energy to destroy tissue. For malignant tumor resection, it may be desirable to preserve tissue architecture to confirm accurate diagnosis and to confirm complete removal and treatment of the tissue. Since tissue architecture may be destroyed during RF energy delivery, there may be a delayed or incomplete medical confirmation of a successful tissue resection. For example, the destroyed tissue architecture may delay or inhibit proper biopsy and classification of the treated tissue. Additionally, RF energy delivery devices may cause postoperative complications and tissue artifact as a result of, for example, delayed tissue effects. Therefore, a need exists for a fast, accurate, and precise method of resection with minimal collateral tissue damage.

US 2006/0206028 A1 discloses an apparatus for ablating undesirable deposits along the inner blood vessel wall of human and animals, the apparatus comprising: a blood extracting and pressurizing unit for extracting source blood from a supply blood vessel and pressurizing the extracted source blood; and a blood delivering and injecting unit, in communicative connection with said blood extracting and pressurizing unit, for delivering and forcefully injecting the pressurized source blood into a blood vessel under treatment, wherein the direction of blood flow is designated as Z-direction of a Cartesian coordinate system, thereby, besides inducing a concomitant blood circulation from said supply blood vessel through said blood vessel under treatment, the apparatus ablates said undesirable deposits from a portion of said blood vessel under treatment in proximity to said blood delivering and injecting unit.

US 2018/0206864 A1 discloses a system including an elongate member comprising a distal end, a proximal end, and at least one fluid delivery lumen extending therebetween; and at least one fluid delivery aperture disposed on a distal portion of the elongate member, wherein the at least one fluid delivery aperture is in fluid communication with the fluid delivery lumen.

US 4,898,574 A discloses a lithotomic apparatus which includes a probe for insertion into a body cavity. A distal tip is fixed to the distal end of the probe and has nozzles. Each nozzle has a rear end communicating with a feed passage defined in the probe and a front end opening to the distal end face of the distal tip. A high-pressure fluid supplied from a source is fed to the feed passage of the probe and pulsatively ejected from the nozzles toward a calculus in the body cavity by a drive mechanism.

### SUMMARY

The claimed invention comprises a medical device as defined by the appended independent claim 1. Further embodiments of the claimed invention are described in the appended dependent claims.

The claimed medical device comprises a body that has a proximal end with a proximal opening. The body defines a channel from the proximal opening to a distal opening configured to emit a fluid jet along a longitudinal axis of the body. The body further includes a distal wall having an inner surface extending in a direction transverse to the longitudinal axis and facing the distal opening to receive the fluid jet. The body defines a space between the distal wall surface and the distal opening. The distal wall includes a protrusion configured to engage tissue, wherein the protrusion provides a solid surface for the fluid jet to impact. The distal opening is configured to emit the fluid jet at a pressure to pierce the tissue and the pressure is at or below 17.2 bar [250 pounds per square inch], and the solid surface provided by the protrusion is convex or concave relative to the flow of the fluid jet.

In some example embodiments, the distal opening may have a diameter of approximately 1 millimeter or less. The proximal opening may have a diameter that is larger than a diameter of the distal opening. The channel may taper in a cross-sectional size from the proximal opening to the distal opening, and the protrusion may comprise one or more pointed tips to engage the tissue.

The body may further comprise a valve and a spring, the valve being fixedly coupled and disposed proximal to the spring and positioned between the proximal opening and the distal opening of the tubular member. The body may be electrically conductive to deliver radio frequency (RF) energy to the tissue. The RF energy delivered to the body may be conductive to the distal wall surface. The body may comprise a bottom surface disposed along the longitudinal axis between the distal opening and the distal wall surface to define a region to engage tissue. The medical device may also comprise a flexible tube coupled to the proximal end of the body. The flexible tube may have a channel to deliver fluid to the body. The flexible tube may include an electrically conductive tube, wire, cable, or braid for delivery of radio frequency (RF) energy to the body.

It may be understood that both the foregoing general description and the following detailed description are exemplary and explanatory only and are not restrictive of the invention, which is defined by the appended set of claims. As used herein, the terms "comprises," "comprising," or any other variation thereof, are intended to cover a non-exclusive inclusion, such that a process, method, article, or apparatus that comprises a list of elements does not include only those elements, but may include other elements not expressly listed or inherent to such process, method, article, or apparatus. The term "exemplary" is used in the sense of "example," rather than "ideal." As used herein, the term "proximal" means a direction closer to an operator, and the term "distal" means a direction further from an operator. Although endoscopy is referenced herein, such reference should not be construed as limiting the possible applications of the disclosed tools. For example, the disclosed tools may be used in procedures such as bronchoscopy, ureteroscopey, colonoscopey, or other procedures within the body.

### BRIEF DESCRIPTION OF THE DRAWINGS

The accompanying drawings, which are incorporated in and constitute a part of this specification, illustrate examples of the present disclosure and together with the description, serve to explain the principles of the disclosure.
FIG. 1 is a perspective view of a medical device performing a fluid-powered tissue dissection, according to an embodiment of the disclosure.
FIGs. 2A and 2B show the distal end of the medical device of FIG. 1.
FIG. 3 shows a cross-sectional view of the medical device of FIG. 1.
FIGs. 4A-4C show a cross-sectional view of another embodiment of a medical device.
FIG. 5 shows a cross-sectional view of yet another embodiment of a medical device according to this disclosure and configured to perform dual fluid-powered tissue dissection and an RF energy delivery to tissue.
FIG. 6 shows an example flow chart depicting operations for performing a fluid-powered tissue dissection.

### DETAILED DESCRIPTION

Tissue dissection, and specifically tissue/tumor resection and removal, may benefit from a medical device that implements a fast, accurate, and precise method of resection with minimal collateral tissue damage. Such medical devices may be more effective and advantageous than medical devices that deliver only radio frequency (RF) energy to perform tissue resection, for example, by preserving tissue architecture for confirmation of medical diagnosis and for effective tissue treatment. In some embodiments, fluid may be delivered to perform tissue dissection techniques (e.g., submucosal dissection). Fluid powered systems may be configured to dissect or resect tissue effectively with high precision and low heat. Additionally, fluid powered resection systems may be combined with RF or other energy delivery technologies to provide coagulation and hemostatic function during tissue resection. In one example, fluid powered tissue resection techniques may lead to reduced blood loss during a surgical procedure, and the low temperature helps preserve tissue and vessel architecture. Resection depth can also be controlled with varying fluid pressure applications. Therefore, aspects of the present disclosure and invention are directed to medical devices with fluid powered tissue resection systems.

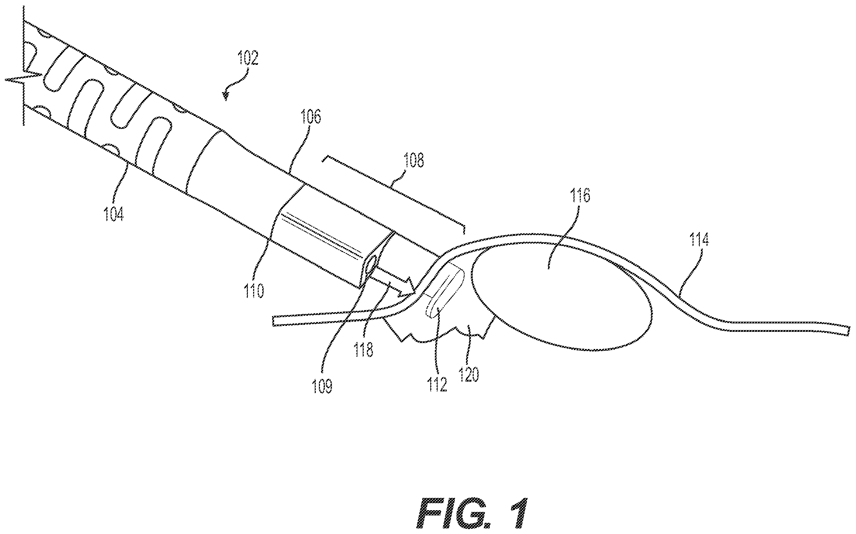

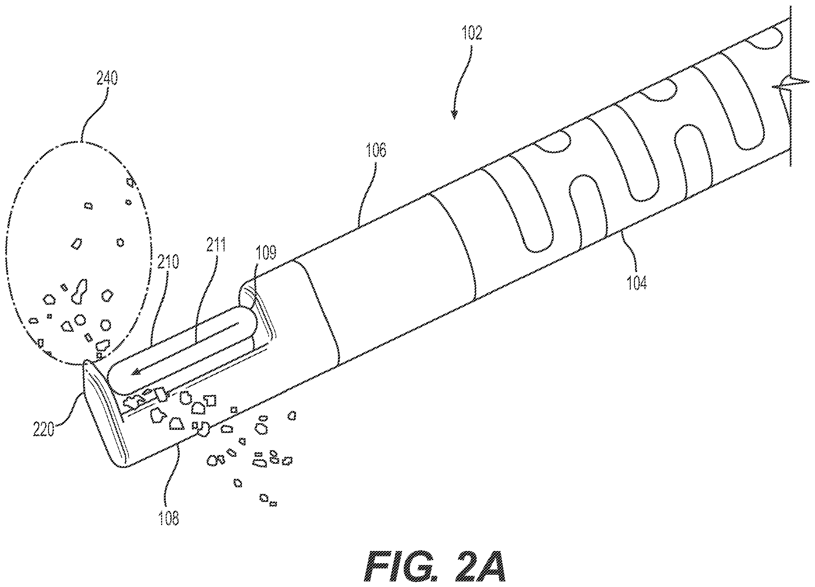

Reference is now made to FIG. 1. FIG. 1 depicts an example medical device 102. The medical device 102 may be, for example, an endoscopic medical device, such as a catheter, used to perform tissue resection methods (e.g., submucosal tissue dissection). The medical device 102 has a tubular member 104 with a proximal end (not shown) and a distal end 106. The tubular member 104 may be any known or contemplated tubular member 104 used for medical procedures, such as endoscopy, and may be, for example, a flexible tubular member with one or more channels or lumens disposed therein and extending between the proximal and distal ends 106 of the tubular member 104 for medical operation. In one example, the tubular member 104 is a catheter, which may be solid, slotted, braided, injection molded, or reflown. As shown in FIG. 1 and FIG. 2A, at least a distal portion of tubular member 104 is slotted to, for example, add flexibility to the tubular member 104. The slotted portion may extend to an unslotted portion at distal end 106, which couples to a body 108 to be described. In one example, the tubular member may be an extruded device. The medical device 102 also has a handle (not shown) connected to the proximal end of the tubular member 104. An operator may use the handle to perform operations of the medical device 102, including those described by the examples herein. The handle may be any known or contemplated handle used for medical procedures, and may include suitable ports and plugs for fluid and/or energy delivery. Additionally, the medical device 102 has a fluid delivery mechanism (not shown) located at the proximal end of the tubular member 104. The fluid delivery mechanism may or may not be part of the handle, and enables fluid to flow from the proximal end of the tubular member 104, via one or more internal channel or lumens, to the distal end 106 and ultimately to a body 108 to perform the fluid powered tissue resection techniques described herein. In one example, the fluid delivery mechanism may include a fluid source that is disposed at the proximal end of the tubular member 104, for example within the handle. In another example, the fluid delivery mechanism may be a mechanism to drive fluid through the tubular member 104 (e.g., a pump) from a remote fluid source.

The medical device 102 also includes a body 108 disposed on the distal end 106 of the tubular member 104. The body 108 has a proximal end 110 and a distal end 112. In one example, the proximal end 110 of the body 108 is configured to interface with/engage the distal end 106 of the tubular member 104. For example, the body 108 may plug into a mating component at the distal end 106 of the tubular member 104. It should be appreciated that any internal working channels and/or lumens may be aligned in the body 108 and the tubular member 104. In other examples, the body 108 may be integrally formed with and permanently fixed to the tubular member 104 (e.g., by being bonded or otherwise adhered or affixed to the tubular member 104). The body 108 has a proximal opening (not shown in FIG. 1) and a distal opening 109.

FIG. 1 also shows a tissue boundary 114. The tissue boundary 114 may be any tissue layer within the human body. FIG. 1 also shows a target tissue at reference numeral 116. In one example, the target tissue 116 may be a tumor located within a gastrointestinal (GI) endothelium, though it should be appreciated that this may be any tissue in the human body. The techniques described herein enable treatment of the target tissue 116, for example, by providing a fluid powered tissue resection method. FIG. 1 shows the distal end 112 of the body 108 embedded below the tissue boundary 114. Reference numeral 118 shows a direction at which fluid may be delivered at a sufficiently high pressure to pierce the tissue boundary 114 and a tissue region 120 where treatment is being applied. Ultimately, the medical device 102 is used to resect the target tissue 116. These systems and methods are described in more detail herein.

Reference is now made to FIG. 2A, which shows the body 108 at the distal end 106 of the tubular member 104 according to one example embodiment. As stated above, the body 108 is configured to interface with the tubular member 104, e.g., by plugging into an opening (not shown in FIG. 1) at the distal end 106 of the tubular member 104. The body 108 has a proximal opening (not shown in FIG. 1) and the distal opening 109 shown in FIG. 1. An interim fluid channel (not shown in FIG. 1) is formed in the body 108 between the proximal opening of the body 108 and the distal opening 109 of the body 108, as described herein. It should be appreciated that, in one example, the interim fluid channel aligns with at least one channel or lumen of the tubular member 104 (e.g., catheter). FIG. 2A shows a fluid jet 210 that is emitted along an axis (e.g., a longitudinal axis) from the distal opening 109 of the body 108. FIG. 2A also shows a direction of fluid flow along the axis, at reference numeral 211. The proximal opening of the body 108 is configured to interface with, and receive fluid from, a fluid delivery device (e.g., the fluid delivery mechanism described in connection with FIG. 1) that is internal and/or external to the medical device 102. The distal opening 109 of the body 108 is configured to emit fluid delivered from the fluid delivery device. An interim fluid channel (not shown in FIG. 2A) is formed in the body 108 between the proximal opening of the body 108 and the distal opening 109 of the body 108, as described herein. In one example, the fluid delivery device may be a fluid lumen or channel disposed within the tubular member 104 of the medical device 102, such that fluid (e.g., water or a saline solution) is delivered from a source at the proximal end of the medical device 102 to the proximal opening of the body 108 for egress through the distal opening 109 of the body 108. In this example, the body 108 is configured to deliver the fluid jet 210 as the fluid is emitted from the fluid delivery device.

The fluid jet 210 may be a fluid jet of water, saline, or other liquid that is delivered at a fluid pressure sufficient for tissue resection. For example, the fluid jet 210 may be emitted through the distal opening 109 of the body 108 at a fluid pressure of up to 60 atmospheric bars ("bars"), or approximately up to 870 pounds per square inch ("psi"). In one embodiment, the fluid jet 210 is emitted at a fluid pressure of 17.2 bar [250 psi] or less when the diameter of the distal opening 109 of the body 108 is 1 millimeters ("mm"). It should be appreciated that the proper fluid pressure may vary depending on system and device parameters, including but not limited to the tissue type, the fluid used for the fluid jet 210, the diameter of the distal opening 109 of the body 108, etc. In one example, the diameter of the distal opening 109 varies based on the channels of the tubular member 104 and the desired size of the intended area of tissue impact of the fluid jet 210. For example, a fluid pressure of between about 20-60 bars may be used for tissue resection, with, e.g., relatively lower pressures providing cleaner and more precise tissue piercings or perforations to minimize risk. In one example, when the distal opening 109 has a diameter of about 1.0 mm [0.04 inches], less than about 17.2 bar [250 psi] of fluid pressure may be sufficient to pierce tissue (e.g., muscle tissue, diseased tissue, or other types of tissue to be treated by the medical device 102), and when the distal opening 109 has a diameter of about 1.3 mm [0.05 inches], less than about 6.9 bar [100 psi] of fluid pressure may be sufficient to pierce tissue. Higher fluid pressures may be desirable for tissue resection of mucosal and/or submucosal layers as opposed to fluid pressures for muscle tissue since the mucosal and submucosal layers of the GI tract may be tougher than muscle. In one example, fluid pressure of about 41.4 bar [600 psi] may pierce mucosal and/or submucosal tissue. The fluid pressure may also vary based on the type of distal opening 109 (e.g., the internal shape and the geometry of the distal opening 109). In one example, the distal opening 109 may be chamfered to disperse pressure, or conical/tapered inward (tapered from a proximal to distal direction) to focus the stream of fluid (e.g., fluid jet 210).

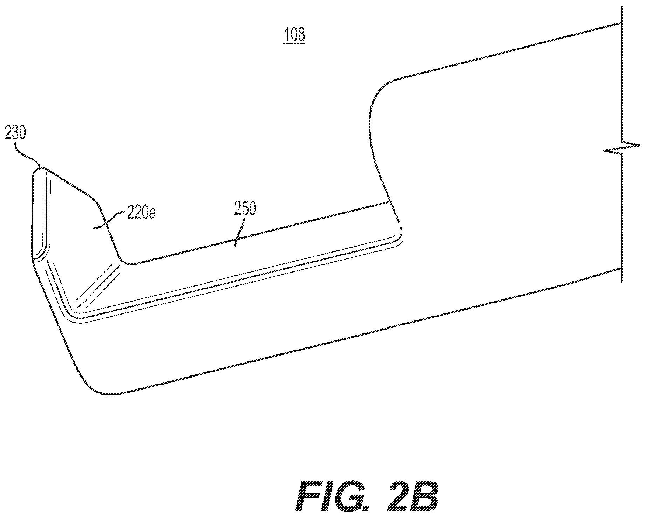

FIG. 2A also shows an outer surface of a distal wall 220. The distal wall 220 extends in a direction transverse to a longitudinal axis along which the fluid jet 210 is emitted. Referring to FIG. 2B, which shows a view of the body 108 according to an example embodiment, an inner, proximally-facing surface of the distal wall 220 is shown at reference numeral 220a. The inner surface 220a faces the distal opening 109 of the body 108. When fluid is delivered through the body 108, the fluid jet 210 is emitted from the distal opening 109 of the body 108 and the fluid jet 210 is received at the inner surface 220a of the distal wall 220. FIG. 2B also shows the distal wall 220 having a protrusion 230 at a top end of the distal wall 220 and extending in the direction transverse to the longitudinal axis at which the fluid jet 210 is emitted. The protrusion 230 may be a relatively sharp tip of the distal wall 220 that points in a radially outward direction. The protrusion 230 is configured to engage tissue (e.g., to pierce the tissue surface and/or be placed on the tissue surface). For example, the protrusion 230 engages the tissue boundary 114, described in connection with FIG. 1, to guide or affix the body 108 to a region proximate a tissue of interest (e.g., the target tissue 116) for ultimate performance of the fluid powered tissue resection methods described herein. In one example, the protrusion 230 is a hook or a hook-like feature that is configured to engage and affix to tissue. The protrusion 230 may comprise one or more tips or prongs.

As the fluid jet 210 is emitted at a sufficiently high fluid pressure to resect tissue, there is a desire to minimize or mitigate unintended tissue perforation. In one example, unmitigated fluid flow without a barrier to block the fluid jet 210 could quickly perforate organs/tissue not intended for tissue treatment. The protrusion 230 may prevent or limit unintended tissue perforation by blocking fluid flow past the tissue that is acquired by the protrusion 230. The protrusion 230 provides a solid surface (e.g., the inner surface 220a of the distal wall 220) for the fluid jet 210 to impact, which may dissipate the force of the fluid jet 210. As the fluid jet 210 impacts the solid surface, the fluid may "splash back." In order to mitigate or prevent unintended tissue perforation from the fluid splash back, the fluid splash back may be at a sufficiently low energy not to cause any damage to the surrounding healthy tissue (and, e.g., to avoid obscuring a camera view of operation). Thus, the protrusion 230 may be shaped to minimize the fluid energy of the splash back as the fluid jet 210 impacts the protrusion 230. According to the claimed invention, the solid surface (e.g., the inner surface 220(a) of the distal wall 220) is convex or concave relative to the flow of the fluid jet 210. In general, the profiles of the distal wall 220 and the protrusion 230 may be optimized in terms of distance from the distal opening 109, shape, material, and thickness in order to direct the splash back safely. Referring back to FIG. 2A, this dissipation is shown at reference numeral 240, as fluid dissipates along the edges and sides of the body 108 and the distal wall 220 and causes minimal or no unintended tissue perforation.

FIG. 2B also shows a bed region ("bed") 250 of the body 108. The bed 250 in one example is a surface disposed along the longitudinal axis between the distal opening 109 of the body 108 and the distal wall 220 of the body 108. The bed 250 may define a space in the body 108 for receiving tissue between the distal opening 109 and the distal wall 220. In one example, the bed 250 is a surface that connects the inner surface 220a of the distal wall 220 to a side of the body 108 with the distal opening 109 of the body 108. The bed 250 may be used during a medical procedure to remove a resected tissue from a patient's body. For example, after the target tissue 116 is treated with the fluid-powered resection methods, the body 108 may be maneuvered such that the target tissue 116 is placed on the bed 250 and removed from the patient's body as the medical device 102 is removed. To assist in capturing the resected tissue and retaining it during retraction of the medical device 102 from the patient, the surface of bed 250 may be treated with a tacky coating or otherwise treated so that the resected tissue adheres to the surface of the bed 250.

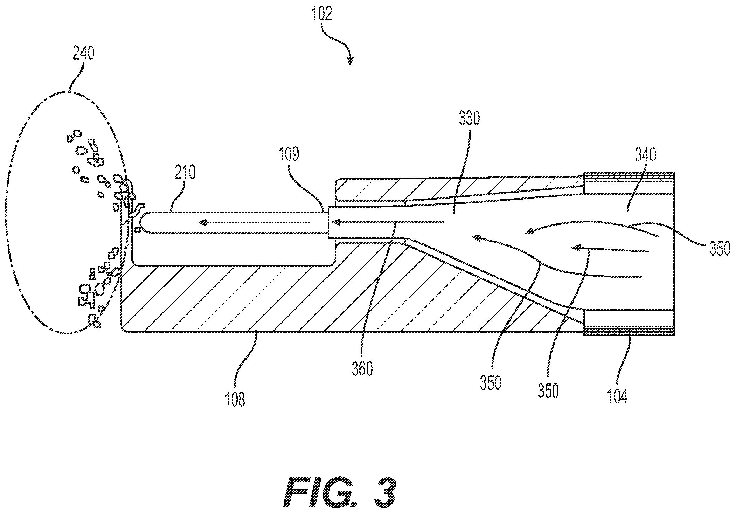

Reference is now made to FIG. 3, which shows a cross-sectional view of the medical device 102. FIG. 3 shows the cross-sectional view of the body 108 and a distal end of the tubular member 104 of the medical device 102. FIG. 3 shows an interim fluid channel 330 in the body 108 and a primary fluid channel 340 in the tubular member 104. As described in connection with FIG. 2A, the interim fluid channel 330 is formed between a proximal opening in the body 108 and the distal opening 109 of the body 108. In FIG. 3, fluid in the primary fluid channel 340 flows to the interim fluid channel 330, as shown by arrows 350, via the proximal opening of the body 108 (not shown in FIG. 3). In other words, the proximal opening of the body 108 interfaces with a distal opening of the primary fluid channel 340 such that fluid may flow between the primary fluid channel 340 and the interim fluid channel 330. The interim fluid channel 330 tapers from a proximal end to the distal end. In other words, the cross-sectional area and/or the diameter of the interim fluid channel 330 decreases from its proximal end to its distal end. As a result, as fluid flows through the interim fluid channel 330 in the distal direction (shown at arrow 360), fluid pressure increases. Thus, the fluid flows at a higher pressure toward the distal end of the interim fluid channel 330 when compared to the fluid flow at the proximal end of the interim fluid channel 330 and when compared to the fluid flow in the primary fluid channel 340. As a result, as the fluid jet 210 egresses from the interim fluid channel 330 at the distal opening 109 of the body 108, the fluid jet 210 is emitted at a higher fluid pressure relative to the fluid pressure of the primary fluid channel 340. In this example, the distal opening 109 of the body 108 operates as a nozzle to emit the fluid jet 210 at a high relative fluid pressure. As described herein, the fluid pressure of the fluid jet 210 is sufficiently high to perform tissue resection operations. Splash back is limited, as shown by the water dissipation at reference 240.

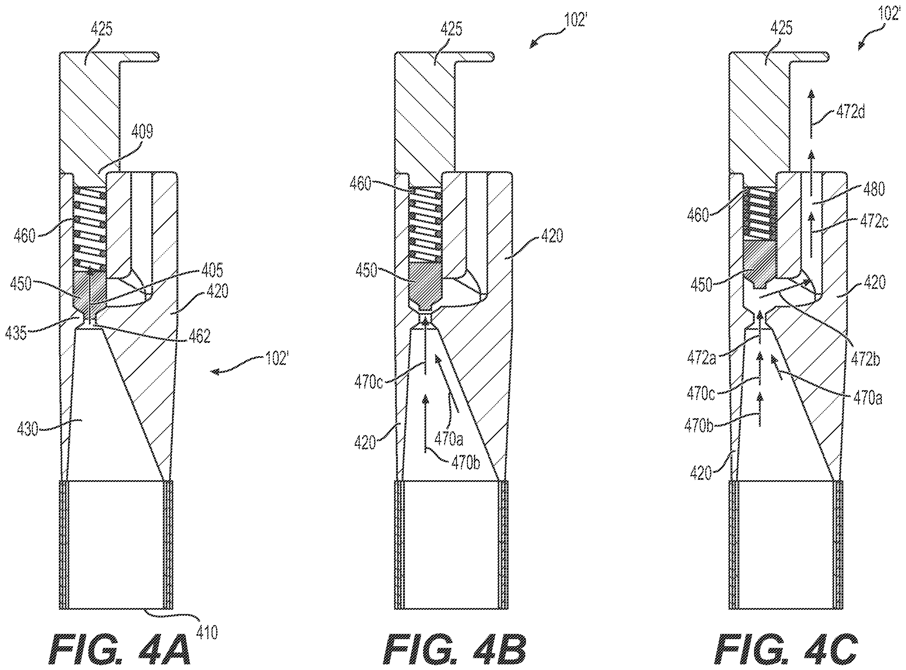

Reference is now made to FIGs. 4A-4C, which show a cross-sectional view of another embodiment a medical device 102'. FIG. 4A shows a tubular member 410, a distal end structure 420, and a body 425. The tubular member 410 may have the structure and functions of the tubular member 104, and the body 425 may have the structure and functions of the body 108. The distal end structure 420 may have the structure and functions of the distal end 106. The distal end structure 420 further includes a valve 450, and a spring 460. In the example depicted in FIG. 4A, fluid may be delivered to a distal end of the medical device 102'. At the distal end, after the fluid has traveled through the tubular member 410 to an interim fluid channel, shown at 430, the interim fluid channel 430 holds the fluid as the interim fluid channel 430 narrows.

The valve 450 is located in a distal portion of the interim fluid channel 430, distal to a narrowing 435 of the interim fluid channel 430. The narrowing 435 has a smaller diameter and/or cross-sectional area relative to portions of channel 430 distal to and proximal to the narrowing 435. The valve 450 is not fixed to the interim fluid channel 430 and is therefore able to translate longitudinally within channel 430, e.g., along a direction shown by arrow 405. The spring 460 also is located in the distal portion of the interim fluid channel 430, distal to the valve 450. The spring 460 is affixed to the valve 450 at a proximal end of the spring 460.

The spring 460 and the valve 450 are positioned between a proximal opening of the interim fluid channel 430, shown at reference 462, and a proximal end of the body 425. In one example, the body 425 is bonded to the distal end structure 420 such that the spring 460 is affixed to a surface of the body 425 at a distal end of the spring 460. In one example, the spring 460 is a coil spring that is compressible upon application of pressure (e.g., upon application of fluid pressure along the direction 405 on the valve 450). In one example, the valve 450 is a one-way valve that allows fluid to egress from the interim fluid channel 430 in a single direction.

Reference is now made to FIGs. 4B and 4C. FIG. 4B shows a closed state configuration of the valve 450. In FIG. 4B, arrows 470a-470c represent fluid flowing into the interim fluid channel 430. The fluid pressure increases at arrow 470c as the interim fluid channel 430 narrows toward the valve 450. In FIG. 4B, the valve is closed, and thus fluid does not egress from the interim fluid channel 430. FIG. 4C shows an open state configuration of the valve 450. In FIG. 4C, once the fluid reaches sufficient pressure (e.g., a threshold pressure), the proximal force exerted by the spring 460 on the valve 450 is overcome, and the pressure forces the valve 450 in a distal direction toward the spring 460 (e.g., along direction 405), thus compressing the spring 460 in the distal direction. Once the distal motion begins, a shape of the valve 450 may result in a greater area of the valve 450 exposed to fluid flow, forcing it to open quickly. Thus, when the spring 460 is in the compressed state (e.g., when the valve 450 and the spring 460 have moved in a distal direction beyond a threshold distance resulting from the threshold pressure), fluid may be able to flow around the valve, as shown in arrows 472a-472d, into an egress channel, shown at reference 480. Egress channel 480 may extend from the proximal opening 462 to the distal end structure 420, such that fluid may flow towards the body 425 e.g., in the direction of arrows 472a-472d. The fluid leaves the egress channel 480 and flows toward the protrusion (e.g., protrusion 230). As fluid pressure in the interim fluid channel 430 decreases (e.g., when the fluid supplied to the device 102' is decreased), the proximal pressure of the spring 460 on the valve 450 may be greater than the fluid pressure exerted on the valve 450, and the valve 450 may recede (e.g., travel in a proximal direction) to the closed state shown in FIG. 4B.

In one example, the fluid is emitted from the interim fluid channel 430 at a constant or relatively constant pressure. Thus, the mechanism described in FIGs. 4A-4C enables fluid to be emitted from the egress channel 480 at a constant or near constant pressure, which avoids a scenario where the fluid pressure gradually builds up during fluid egress from the interim fluid channel 430 and the distal opening 462. In some examples, it is advantageous for fluid to be emitted from the egress channel 480 at a substantially constant pressure to avoid unintended tissue damage during pressure build up or suboptimal tissue resection.

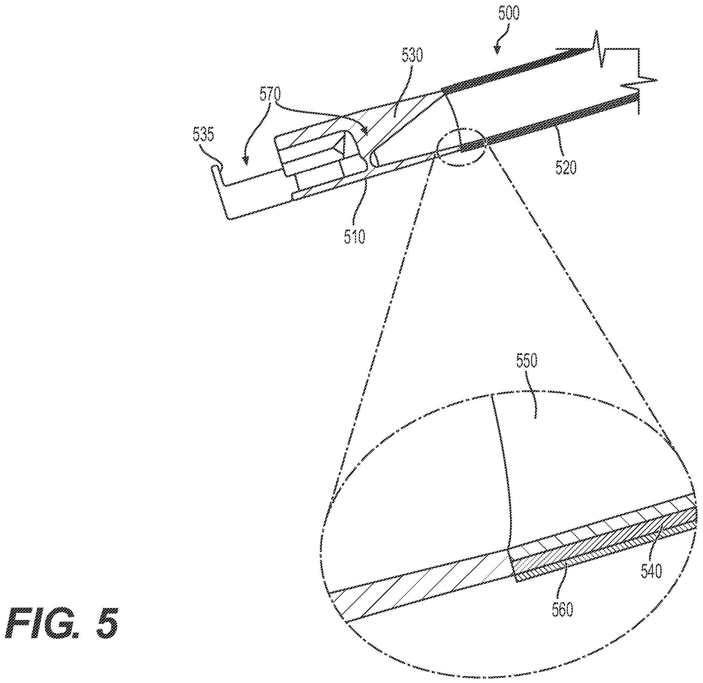

Reference is now made to FIG. 5. FIG. 5 shows a cross-sectional view of yet another embodiment of a medical device at 500. In general, the device 500 is configured for fluid powered tissue dissection and RF energy delivery to tissue. FIG. 5 shows the cross-sectional view of tubular member 520 and body 530. The tubular member 520 and the body 530 can have any of the structure and functions of tubular member 104, 420 and the body 108, 410, respectively. The medical device 500 is advantageous for endoscopic procedures to provide coagulative functions with RF delivery in addition to the tissue resection functions performed by the fluid powered system. The body 530 may be bonded or otherwise affixed to the tubular member 520. The body 530 is made of metal or other electrically conductive material. The body 530 is configured to conduct RF energy. For example, RF energy may be delivered to the body 530 via a conductive tube, wire, cable, or braid of the medical device 500. A conductive tube is shown at reference numeral 540 in the expanded view of the junction between the body 530 and the tubular member 520. The conductive tube 540 may be surrounded by insulating material, shown at reference numerals 550 (an inner insulation) and 560 (an outer insulation). Thus, the conductive tube 540 forms an electrical conduit to carry the RF energy to the body 530 and ultimately to distal wall 535 to coagulate tissue during a medical operation. The RF active components of the body 530 therefore includes the distal wall 535. Thus, in one example, the distal wall 535 may operate as the point of contact to the tissue to deliver RF energy for coagulation.

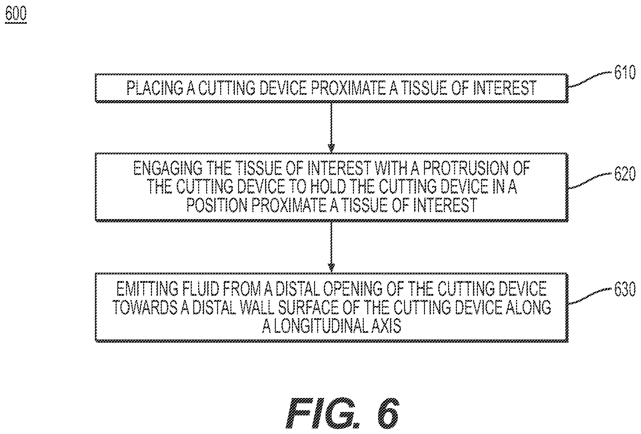

Reference is now made to FIG. 6, which shows an example flow chart 600 depicting operations for performing the fluid-powered tissue resection techniques, described herein. At operation 610, a resection device is placed proximate a tissue of interest. The resection device may be any of the medical devices 102, 102', 500 described herein. At operation 620, the tissue of interest is engaged with a protrusion of the resection device to hold the resection device in a position at the tissue of interest. At operation 630, fluid is emitted from a distal opening of the resection device towards a distal wall surface of the resection device along a longitudinal axis. The fluid resects the tissue of interest.

Other embodiments of the disclosure will be apparent to those skilled in the art from consideration of the specification and practice of the invention disclosed herein. It is intended that the specification and examples be considered as exemplary only, with the true scope of the invention being indicated by the following claims.

It should be understood that one or more of the aspects of any of the medical devices described herein may be used in combination with any other medical device known in the art, such as medical imaging systems or other scopes such as colonoscopes, bronchoscopes, ureteroscopes, duodenoscopes, etc., or other types of imagers.

It also should also be understood that one or more aspects of any of the medical devices described herein may be used for resection, cutting, or otherwise dissecting tissue in any part of the human body. For example, any of the medical devices described herein may be used in medical procedures where removal and/or detection of tissue is needed.

While principles of the present disclosure are described herein with reference to illustrative examples for particular applications, it should be understood that the disclosure is not limited thereto. Accordingly, the invention is not to be considered as limited by the foregoing description but is defined by the following set of claims.

## Claims

1. A medical device (102) comprising:
a body (108) having a proximal end (110) with a proximal opening, the body defining a channel from the proximal opening to a distal opening (109) configured to emit a fluid jet (210) along a longitudinal axis of the body;
the body further including a distal wall (220) having an inner surface (220a) extending in a direction transverse to the longitudinal axis and facing the distal opening to receive the fluid jet, the body defining a space between the distal wall surface and the distal opening, the distal wall including a protrusion (230) configured to engage tissue, wherein the protrusion provides a solid surface for the fluid jet to impact;
wherein
the distal opening is configured to emit the fluid jet at a pressure to pierce the tissue and **characterized in that** the pressure is at or below 17.2 bar (250 pounds per square inch), and
the solid surface provided by the protrusion is convex or concave relative to the flow of the fluid jet.

2. The medical device of claim 1, wherein the distal opening has a diameter of approximately 1 millimeter or less.

3. The medical device of claim any of the preceding claims, wherein the proximal opening has a diameter that is larger than a diameter of the distal opening.

4. The medical device of claim 3, wherein the channel tapers in cross-sectional size from the proximal opening to the distal opening.

5. The medical device of any of the preceding claims, wherein the protrusion comprises one or more pointed tips to engage the tissue.

6. The medical device of any of the preceding claims, wherein the body receives a fluid at the proximal opening and further comprises a valve (450) and a spring (460) configured to maintain the fluid within the body until a pressure of the fluid is above a predetermined threshold.

7. The medical device of claim 6, wherein the valve is fixedly coupled to the spring, the valve and the spring are positioned between the proximal opening and the distal opening, and the valve is proximal to the spring.

8. The medical device of claim 6 or 7, wherein the valve is a one way valve, and the spring is a coil spring having a distal end fixed within the body.

9. The medical device of any of the preceding claims, wherein the body is electrically conductive to deliver radio frequency, RF, energy to the tissue.

10. The medical device of claim 9, wherein the RF energy delivered to the body is conductive to the distal wall surface.

11. The medical device of any of the preceding claims, wherein the body further comprises a bottom surface disposed along the longitudinal axis between the distal opening and the distal wall to define a region to engage tissue.

12. The medical device of any of the preceding claims, further comprising a flexible tube (104, 520) coupled to the proximal end of the body, the flexible tube including a channel to deliver a fluid to the body.

13. The medical device of claim 12, wherein the flexible tube includes an electrically conductive tube (540), wire, cable, or braid for delivery of radio frequency, RF, energy to the body.

## Patentansprüche

1. Medizinische Vorrichtung (102), aufweisend:
einen Körper (108) mit einem proximalen Ende (110) mit einer proximalen Öffnung, wobei der Körper einen Kanal von der proximalen Öffnung zu einer distalen Öffnung (109) definiert, die dafür konfiguriert ist, einen Fluidstrahl (210) entlang einer Längsachse des Körpers auszustoßen,
wobei der Körper ferner eine distale Wand (220) mit einer inneren Oberfläche (220a) beinhaltet, die sich in einer Richtung quer zur Längsachse erstreckt und der distalen Öffnung zugewandt ist, um den Fluidstrahl zu empfangen, wobei der Körper einen Raum zwischen der distalen Wandoberfläche und der distalen Öffnung definiert, wobei die distale Wand einen Vorsprung (230) beinhaltet, der dafür konfiguriert ist, mit Gewebe in Eingriff zu treten, wobei der Vorsprung eine feste Oberfläche bereitstellt, auf die der Fluidstrahl auftreffen kann,
wobei die distale Öffnung dafür konfiguriert ist, den Fluidstrahl mit einem Druck auszustoßen, dass er das Gewebe durchsticht, und
**dadurch gekennzeichnet, dass**
der Druck bei oder unter 17.2 bar (250 Pfund pro Quadratzoll) liegt, und
die durch den Vorsprung bereitgestellte feste Oberfläche relativ zur Strömung des Fluidstrahls konvex oder konkav ist.

2. Medizinische Vorrichtung nach Anspruch 1, wobei die distale Öffnung einen Durchmesser von etwa 1 Millimeter oder weniger hat.

3. Medizinische Vorrichtung nach irgendeinem der vorhergehenden Ansprüche, wobei die proximale Öffnung einen Durchmesser hat, der größer ist als ein Durchmesser der distalen Öffnung.

4. Medizinische Vorrichtung nach Anspruch 3, wobei sich der Kanal in der Querschnittsgröße von der proximalen Öffnung zur distalen Öffnung verjüngt.

5. Medizinische Vorrichtung nach irgendeinem der vorhergehenden Ansprüche, wobei der Vorsprung eine oder mehrere spitze Enden aufweist, um mit dem Gewebe in Eingriff zu treten.

6. Medizinische Vorrichtung nach irgendeinem der vorhergehenden Ansprüche, wobei der Körper ein Fluid an der proximalen Öffnung empfängt und ferner ein Ventil (450) und eine Feder (460) aufweist, die dafür konfiguriert sind, das Fluid innerhalb des Körpers zu halten, bis ein Druck des Fluids einen vorbestimmten Schwellenwert überschreitet.

7. Medizinische Vorrichtung nach Anspruch 6, wobei das Ventil fest mit der Feder verbunden ist, das Ventil und die Feder zwischen der proximalen Öffnung und der distalen Öffnung positioniert sind, und das Ventil proximal zur Feder ist.

8. Medizinische Vorrichtung nach Anspruch 6 oder 7, wobei das Ventil ein Einwegventil ist, und die Feder eine Schraubenfeder mit einem innerhalb des Körpers fixierten distalen Ende ist.

9. Medizinische Vorrichtung nach irgendeinem der vorhergehenden Ansprüche, wobei der Körper elektrisch leitfähig ist, um dem Gewebe Hochfrequenz-, HF-, Energie zuzuführen.

10. Medizinische Vorrichtung nach Anspruch 9, wobei die dem Körper zugeführte HF-Energie zur distalen Wandoberfläche geleitet werden kann.

11. Medizinische Vorrichtung nach irgendeinem der vorhergehenden Ansprüche, wobei der Körper ferner eine untere Oberfläche aufweist, die entlang der Längsachse zwischen der distalen Öffnung und der distalen Wand angeordnet ist, um einen Bereich zu definieren, um mit Gewebe in Eingriff zu treten.

12. Medizinische Vorrichtung nach irgendeinem der vorhergehenden Ansprüche, ferner aufweisend ein flexibles Rohr (104, 520), das mit dem proximalen Ende des Körpers gekoppelt ist, wobei das flexible Rohr einen Kanal zum Zuführen eines Fluids an den Körper beinhaltet.

13. Medizinische Vorrichtung nach Anspruch 12, wobei das flexible Rohr ein elektrisch leitfähiges Rohr (540), Draht, Kabel oder Geflecht zur Zuführung von Hochfrequenz-, HF-, Energie an den Körper beinhaltet.

## Revendications

1. Dispositif médical (102) comprenant:
un corps (108) ayant une extrémité proximale (110) avec une ouverture proximale, le corps définissant un canal depuis l'ouverture proximale jusqu'à une ouverture distale (109) configurée pour émettre un jet de fluide (210) le long d'un axe longitudinal du corps;
le corps incluant en outre une paroi distale (220) ayant une surface interne (220a) s'étendant dans une direction transversale à l'axe longitudinal et faisant face à l'ouverture distale pour recevoir le jet de fluide, le corps définissant un espace entre la surface de la paroi distale et l'ouverture distale, la paroi distale incluant une saillie (230) configurée pour venir en contact avec un tissu, dans lequel la saillie fournit une surface solide destinée à être frappée par le jet de fluide;
dans lequel l'ouverture distale est configurée pour émettre le jet de fluide à une pression permettant de percer le tissu et **caractérisé en ce que** la pression est égale ou inférieure à 17,2 bars (250 livres par pouce carré), et
la surface solide fournie par la saillie est convexe ou concave par rapport à l'écoulement du jet de fluide.

2. Dispositif médical selon la revendication 1, dans lequel l'ouverture distale a un diamètre d'approximativement 1 millimètre ou moins.

3. Dispositif médical selon l'une quelconque des revendications précédentes, dans lequel l'ouverture proximale a un diamètre qui est supérieur à un diamètre de l'ouverture distale.

4. Dispositif médical selon la revendication 3, dans lequel le canal se rétrécit en taille de section transversale depuis l'ouverture proximale jusqu'à l'ouverture distale.

5. Dispositif médical selon l'une quelconque des revendications précédentes, dans lequel la saillie comprend une ou plusieurs pointes pointues pour venir en contact avec le tissu.

6. Dispositif médical selon l'une quelconque des revendications précédentes, dans lequel le corps reçoit un fluide au niveau de l'ouverture proximale et comprend en outre une vanne (450) et un ressort (460) configurés pour maintenir le fluide à l'intérieur du corps jusqu'à ce qu'une pression du fluide soit supérieure à un seuil prédéterminé.

7. Dispositif médical selon la revendication 6, dans lequel la vanne est couplée de manière fixe au ressort, la vanne et le ressort sont positionnés entre l'ouverture proximale et l'ouverture distale, et la vanne est proximale par rapport au ressort.

8. Dispositif médical selon la revendication 6 ou 7, dans lequel la vanne est une vanne unidirectionnelle et le ressort est un ressort hélicoïdal ayant une extrémité distale fixée à l'intérieur du corps.

9. Dispositif médical selon l'une quelconque des revendications précédentes, dans lequel le corps est électriquement conducteur pour délivrer une énergie radiofréquence, RF, au tissu.

10. Dispositif médical selon la revendication 9, dans lequel l'énergie RF délivrée au corps est conduite à la surface de la paroi distale.

11. Dispositif médical selon l'une quelconque des revendications précédentes, dans lequel le corps comprend en outre une surface inférieure disposée le long de l'axe longitudinal entre l'ouverture distale et la paroi distale pour définir une région pour venir en contact avec le tissu.

12. Dispositif médical selon l'une quelconque des revendications précédentes, comprenant en outre un tube flexible (104, 520) couplé à l'extrémité proximale du corps, le tube flexible incluant un canal pour délivrer un fluide au corps.

13. Dispositif médical selon la revendication 12, dans lequel le tube flexible inclut un tube électriquement conducteur (540), un fil, un câble ou une tresse pour délivrer de l'énergie radiofréquence, RF, au corps.
